# EUROPÄISCHE PATENTSCHRIFT

(11) **EP 3 066 097 B1**
(45) Veröffentlichungstag und Bekanntmachung des Hinweises auf die Patenterteilung: **16.08.2017**
(21) Anmeldenummer: 14793565.4
(22) Anmeldetag: 05.11.2014
(51) Int. Cl.: C07D 413/04, A61K 31/513, A61P 9/00

(54) **SALZE VON 1-(3-METHYL-2-OXO-2,3-DIHYDRO-1,3-BENZOXAZOL-6-YL)-2,4-DIOXO-3-[(1R)-4-(TRIFLUORMETHYL)-2,3-DIHYDRO-1H-INDEN-1-YL]-1,2,3,4-TETRAHYDROPYRIMIDIN-5-CARBONSÄURE**
SALTS OF 1-(3-METHYL-2-OXO-2,3-DIHYDRO-1,3-BENZOXAZOL-6-YL)-2,4-DIOXO-3-[(1R)-4-(TRIFLUORMETHYL)-2,3-DIHYDRO-1H-INDEN-1-YL]-1,2,3,4-TETRAHYDROPYRIMIDIN-5-CARBONIC ACID
SELS DE 1-(3-METHYL-2-OXO-2,3-DIHYDRO-1,3-BENZOXAZOL-6-YL)-2,4-DIOXO-3-[(1R)-4-(TRIFLUORMETHYL)-2,3-DIHYDRO-1H-INDEN-1-YL]-1,2,3,4-TETRAHYDROPYRIMIDIN-5-ACIDE CARBONIQUE

(30) Priorität: 08.11.2013 EP 13192177
(43) Veröffentlichungstag der Anmeldung: 14.09.2016
(73) Patentinhaber: Bayer Pharma Aktiengesellschaft, 13353 Berlin (DE)
(72) Erfinder: OLENIK, Britta, 46242 Bottrop (DE); KEIL, Birgit, 40231 Düsseldorf (DE); HINZ, Martin-Holger, 42499 Hückeswagen (DE); FÜRSTNER, Chantal, 45478 Mülheim/Ruhr (DE); JESKE, Mario, 42699 Solingen (DE); ACKERSTAFF, Jens, 40225 Düsseldorf (DE)
(74) Vertreter: BIP Patents
(86) Internationale Anmeldenummer: PCT/EP2014/073801
(87) Internationale Veröffentlichungsnummer: WO 2015/067652

(56) Entgegenhaltungen:
- WO-A1-2013/167495
- US-A1- 2004 019 068

## Beschreibung

Die Erfindung betrifft neue Salze von 1-(3-Methyl-2-oxo-2,3-dihydro-1,3-benzoxazol-6-yl)-2,4-dioxo-3-[(1R)-4-(trifluormethyl)-2,3-dihydro-1H-inden-1-yl]-1,2,3,4-tetrahydropyrimidin-5-carbonsäure der Formel (I), insbesondere Aminosäuresalze wie das Lysinsalz und Alkalimetallsalze wie das Natriumsalz und das Kaliumsalz, Verfahren zu deren Herstellung, diese enthaltende Arzneimittel sowie deren Verwendung bei der Bekämpfung von Krankheiten.

1-(3-Methyl-2-oxo-2,3-dihydro-1,3-benzoxazol-6-yl)-2,4-dioxo-3-[(1R)-4-(trifluormethyl)-2,3-dihydro-1H-in-den-1-yl]-1,2,3,4-tetrahydropyrimidin-5-carbonsäure, deren Herstellung und deren Verwendung als Chymase-Inhibitor wird in der Patentanmeldung PCT/EP2013/059286 beschrieben (siehe Beispiel 189) und entspricht der Verbindung der Formel (I):

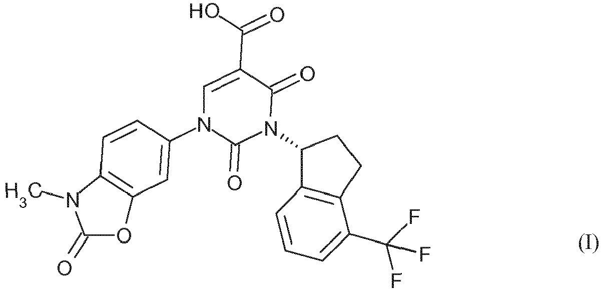

Die Verbindung der Formel (I) wird im Folgenden als freie Säure bezeichnet.

Es wurde nun gefunden, dass die freie Säure für einige Anwendungen eine unzureichende Löslichkeit zeigt und deshalb nicht uneingeschränkt für die Verwendung in Formulierungen geeignet ist.

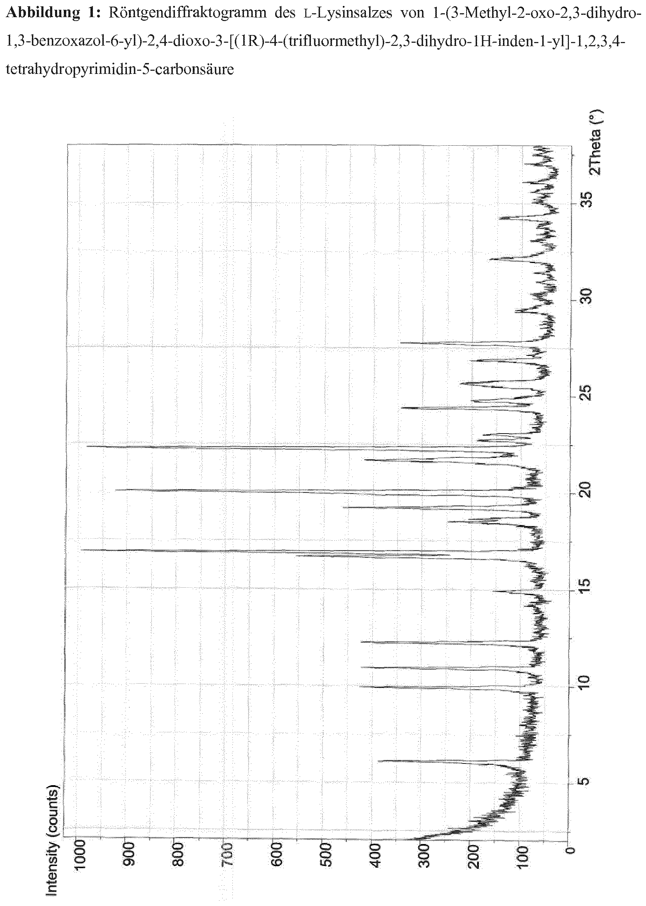

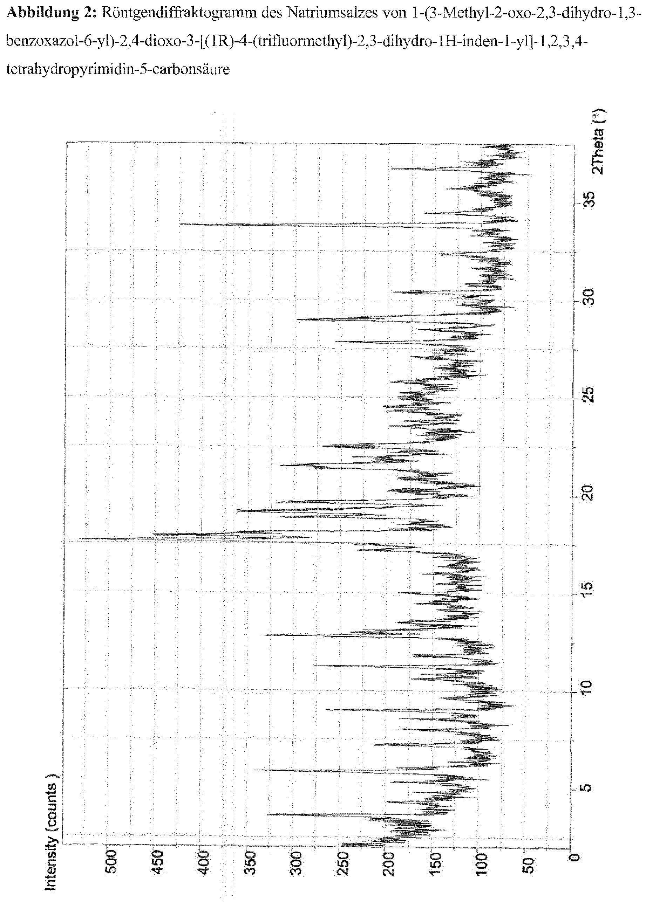

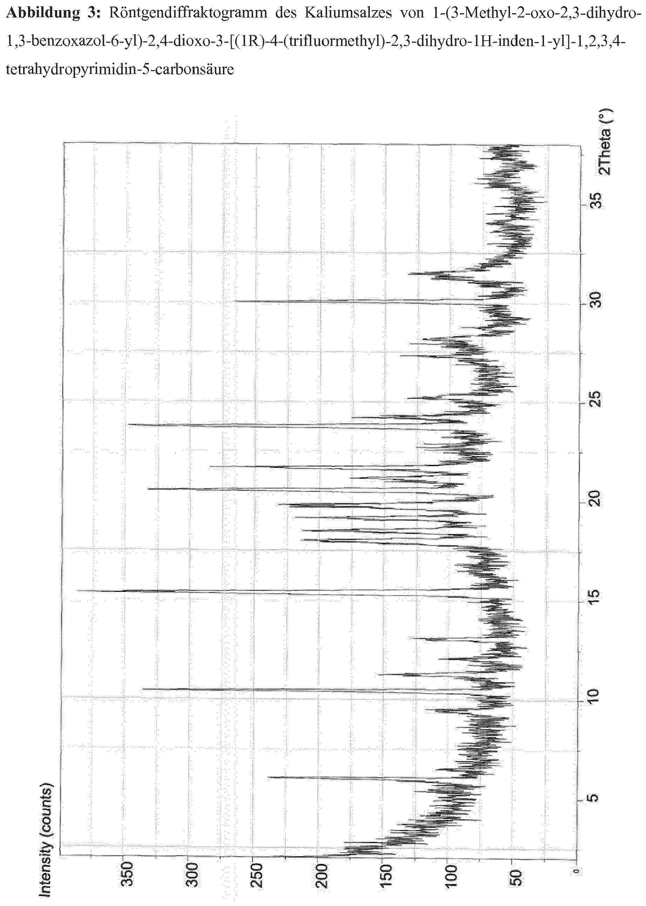

Überraschendeweise wurden nun neue Salze gefunden. Diese Salze haben deutlich unterschiedliche und jeweils charakteristische Röntgendiffraktogramme (Tabelle 1, Abbildung 1, 2 und 3).

Gegenstand der vorliegenden Erfindung ist die Verbindung der Formel (I) in Form ihrer Aminosäuresalze und Alkalimetallsalze.

Gegenstand der vorliegenden Erfindung ist die Verbindung der Formel (I) in Form ihres Lysinsalzes, insbesondere in Form ihres L-Lysinsalzes, bzw. in Form ihres Natriumsalzes oder Kaliumsalzes.

Gegenstand der Erfindung ist die Verbindung der Formel (I) in Form ihres L-Lysinsalzes, das im Röntgendiffraktogramm im Wesentlichen das folgende bevorzugte Peakmaximum des 2 Theta Winkels bei 16.9 aufweist. Ein bevorzugter Gegenstand der Erfindung ist die Verbindung der Formel (I) in Form ihres L-Lysinsalzes, das im Röntgendiffraktogramm im Wesentlichen die folgenden bevorzugten Peakmaxima des 2 Theta Winkels bei 16.9, 22.3 und 20.0 aufweist.

Ein weiterer bevorzugter Gegenstand der Erfindung ist die Verbindung der Formel (I) in Form ihres L-Lysinsalzes, das im Röntgendiffraktogramm im Wesentlichen die folgenden bevorzugten Peakmaxima des 2 Theta Winkels bei 16.9, 22.3, 20.0, 16.7, 19.2, 10.9 und 12.2 aufweist.

Ein weiterer bevorzugter Gegenstand der Erfindung ist die Verbindung der Formel (I) in Form ihres L-Lysinsalzes, das im Röntgendiffraktogramm im Wesentlichen die folgenden bevorzugten Peakmaxima des 2 Theta Winkels bei 16.9, 22.3, 20.0, 16.7, 19.2,, 10.9, 12.2, 9.9 und 21.6 aufweist.

Gegenstand der Erfindung ist außerdem die Verbindung der Formel (I) in Form ihres Natriumsalzes, das im Röntgendiffraktogramm im Wesentlichen das folgende bevorzugte Peakmaximum des 2 Theta Winkels bei 17.6 aufweist.

Ein bevorzugter Gegenstand der Erfindung ist die Verbindung der Formel (I) in Form ihres Natriumsalzes, das im Röntgendiffraktogramm im Wesentlichen die folgenden bevorzugten Peakmaxima des 2 Theta Winkels bei 17.6, 17.9 und 19.1 aufweist.

Ein weiterer bevorzugter Gegenstand der Erfindung ist die Verbindung der Formel (I) in Form ihres Natriumsalzes, das im Röntgendiffraktogramm im Wesentlichen die folgenden bevorzugten Peakmaxima des 2 Theta Winkels bei 17.6, 17.9, 19.1, 18.1, 12.8, 5.9, und 18.9 aufweist.

Ein weiterer bevorzugter Gegenstand der Erfindung ist die Verbindung der Formel (I) in Form ihres Natriumsalzes, das im Röntgendiffraktogramm im Wesentlichen die folgenden bevorzugten Peakmaxima des 2 Theta Winkels bei 17.6, 17.9, 19.1, 18.1, 12.8, 5.9,, 18.9, 29.0 und 19.6 aufweist.

Gegenstand der Erfindung ist außerdem die Verbindung der Formel (I) in Form ihres Kaliumsalzes, das im Röntgendiffraktogramm im Wesentlichen das folgende bevorzugte Peakmaximum des 2 Theta Winkels bei 23.7 aufweist.

Ein bevorzugter Gegenstand der Erfindung ist die Verbindung der Formel (I) in Form ihres Kaliumsalzes, das im Röntgendiffraktogramm im Wesentlichen die folgenden bevorzugten Peakmaxima des 2 Theta Winkels bei 23.7, 15.3und 20.5 aufweist.

Ein weiterer bevorzugter Gegenstand der Erfindung ist die Verbindung der Formel (I) in Form ihres Kaliumsalzes, das im Röntgendiffraktogramm im Wesentlichen die folgenden bevorzugten Peakmaxima des 2 Theta Winkels bei 23.7, 15.3,, 20.5, 10.4, , 30.0, 21.7und 6.0 aufweist.

Ein weiterer bevorzugter Gegenstand der Erfindung ist die Verbindung der Formel (I) in Form ihres Kaliumsalzes, das im Röntgendiffraktogramm im Wesentlichen die folgenden bevorzugten Peakmaxima des 2 Theta Winkels bei 23.7, 15.3,, 20.5, 10.4,, 30.0, 21.7, 6.0, 19.8 und 18.0 aufweist.

Generelle Aspekte im Zusammenhang mit der vorliegenden Erfindung sind pharmakologische Eigenschaften, die Prozessierbarkeit, das Herstellungsverfahren, das Nebenwirkungsprofil, die Stabilität und die pharmakologische Wirksamkeit insbesondere des Salzes mit L-Lysin der Verbindung der Formel (I).

Überraschenderweise sind das L-Lysinsalz sowie das Natrium- und Kaliumsalz der Verbindung der Formel (I) kristallin und auch nach Verarbeitung über Suspensionen lagerstabil. Sie eignet sich deshalb besonders für den Einsatz in pharmazeutischen Formulierungen, wie z. B. Suspensionen oder Cremes, aber auch in anderen Zubereitungen, die über suspendierten Wirkstoff hergestellt werden, wie beispielsweise bei der wässrigen Granulation oder Nassmahlung.

Die erfindungsgemäßen Salze der Verbindung der Formel (I), insbesondere das L-Lysinsalz sowie das Natrium- und Kaliumsalz, werden in pharmazeutischen Formulierungen in hoher Reinheit eingesetzt. Aus Stabilitätsgründen enthält eine pharmazeutische Formulierung hauptsächlich ein Salz der Verbindung der Formel (I), insbesondere entweder das L-Lysinsalz oder das Natrium- oder Kaliumsalz, und keine größeren Anteile einer anderen Form der Verbindung der Formel (I). Bevorzugt enthält das Arzneimittel mehr als 90 Gewichtsprozente, besonders bevorzugt mehr als 95 Gewichtsprozente der Verbindung der Formel (I) in Form des entsprechenden Salzes bezogen auf die Gesamtmenge der enthaltenen Verbindung der Formel (I).

Die erfindungsgemäßen Salze besitzen wertvolle pharmakologische Eigenschaften und können zur Behandlung und/ oder Prophylaxe von Erkrankungen bei Menschen und Tieren verwendet werden. Die erfindungsgemäßen Salze stellen Inhibitoren der Chymase dar und eignen sich daher zur Behandlung und/oder Prophylaxe kardiovaskulärer, entzündlicher, allergischer und/oder fibrotischer Erkrankungen.

Im Sinne der vorliegenden Erfindung sind unter Erkrankungen des Herzkreislauf-Systems beziehungsweise kardiovaskulären Erkrankungen beispielsweise die folgenden Erkrankungen zu verstehen: akute und chronische Herzinsuffizienz, arterielle Hypertonie, koronare Herzerkrankung, stabile und instabile Angina pectoris, myokardiale Ischämie, Myokardinfarkt, Schock, Atherosklerose, Herzhypertrophie, Herzfibrose, atriale und ventrikuläre Arrhythmien, transitorische und ischämische Attacken, Hirnschlag, Präeklampsie, entzündliche kardiovaskuläre Erkrankungen, periphere und kardiale Gefäßerkrankungen, periphere Durchblutungsstörungen, arterielle pulmonale Hypertonie, Spasmen der Koronararterien und peripherer Arterien, Thrombosen, thromboembolische Erkrankungen, Ödembildung wie zum Beispiel pulmonales Ödem, Hirnödem, renales Ödem oder Herzinsuffizienz-bedingtes Ödem, sowie Restenosen wie nach Thrombolysetherapien, percutan-transluminalen Angioplastien (PTA), transluminalen Koronarangioplastien (PTCA), Herztransplantationen und Bypass-Operationen, sowie mikro- und makrovaskuläre Schädigungen (Vasculitis), Reperfusionsschäden, arterielle und venöse Thrombosen, Mikroalbuminurie, Herzmuskelschwäche, endotheliale Dysfunktion, periphere und kardiale Gefäßerkrankungen, periphere Durchblutungsstörungen, Herzinsuffizienz-bedingtes Ödem, erhöhte Spiegel von Fibrinogen und von LDL geringer Dichte sowie erhöhte Konzentrationen von Plasminogenaktivator-Inhibitor 1 (PAI-1).

Im Sinne der vorliegenden Erfindung umfasst der Begriff Herzinsuffizienz auch spezifischere oder verwandte Krankheitsformen wie akut dekompensierte Herzinsuffizienz, Rechtsherzinsuffizienz, Linksherzinsuffizienz, Globalinsuffizienz, ischämische Kardiomyopathie, dilatative Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz.

Die erfindungsgemäßen Salze sind weiter geeignet für die Prophylaxe und/oder Behandlung der polyzystischen Nierenkrankheit (PCKD) und des Syndroms der inadäquaten ADH-Sekretion (SIADH). Weiterhin eignen sich die erfindungsgemäßen Salze zur Behandlung und/oder Prophylaxe von Nierenerkrankungen, insbesondere von aktuer und chronischer Niereninsuffizienz, sowie von akutem und chronischem Nierenversagen.

Im Sinne der vorliegenden Erfindung umfasst der Begriff akute Niereninsuffizienz akute Erscheinungsformen der Nierenerkrankung, des Nierenversagens und/ oder der Niereninsuffizienz mit und ohne Dialysepflicht, wie auch zugrundeliegende oder verwandte Nierenerkrankungen wie renale Hypoperfusion, intradialytische Hypotonie, Volumenmangel (z.B. Dehydratation, Blutverlust), Schock, akute Glomerulonephritis, hämolytischurämisches Syndrom (HUS), vaskuläre Kathastrophe (arterielle oder venöse Thrombose oder Embolie), Cholesterinembolie, akute Bence-Jones-Niere bei Plasmozytom, akute supravesikal oder subvesikale Abflussbehinderungen, immunlogische Nierenerkrankungen wie Nierentransplantatabstoßung, Immunkomplex-induzierte Nierenerkrankungen, tubuläre Dilatation, Hyperphosphatämie und/ oder akute Nierenerkrankungen, die durch die Notwendigkeit zur Dialyse charakterisiert werden können, sowie bei Teilresektionen der Niere, Dehydratation durch forcierte Diurese, unkontrolliertem Blutdruckanstieg mit maligner Hypertonie, Harnwegsobstruktion und -infekt und Amyloidose sowie Systemerkrankungen mit glomerulärer Beteiligung, wie rheumatologischinmmnologische Systemerkrankungen, wie beispielsweise Lupus erythematodes, Nierenarterienthrombose, Nierenvenenthrombose, Analgetikanephropathie und renal-tubuläre Azidose, sowie Röntgen-Kontrastmittelsowie Medikamenten-induzierte akute interstitielle Nierenerkrankungen.

Im Sinne der vorliegenden Erfindung umfasst der Begriff chronische Niereninsuffizienz chronische Erscheinungsformen der Nierenerkrankung, des Nierenversagens und/ oder der Niereninsuffizienz mit und ohne Dialysepflicht, wie auch zugrundeliegende oder verwandte Nierenerkrankungen wie renale Hypoperfusion, intradialytische Hypotonie, obstruktive Uropathie, Glomerulopathien, glomeruläre und tubuläre Proteinurie, renale Ödeme, Hämaturie, primäre, sekundäre sowie chronische Glomerulonephritis, membranöse und membranoproliferative Glomerulonephritis, Alport-Syndrom, Glomerulosklerose, tubulointerstitielle Erkrankungen, nephropathische Erkrankungen wie primäre und angeborene Nierenerkrankung, Nierenentzündung, immunlogische Nierenerkrankungen wie Nierentransplantatabstoßung, Immunkomplex-induzierte Nierenerkrankungen, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/ oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/ oder die Notwendigkeit zur Dialyse charakterisiert werden können, sowie bei Nierenzellkarzinomen, nach Teilresektionen der Niere, Dehydratation durch forcierte Diurese, unkontrollierter Blutdruckanstieg mit maligner Hypertonie, Harnwegsobstruktion und -infekt und Amyloidose sowie Systemerkrankungen mit glomerulärer Beteiligung, wie rheumatologisch-immunologische Systemerkrankungen, wie beispielsweise Lupus erythematodes, sowie Nierenarterienstenose, Nierenarterienthrombose, Nierenvenenthrombose, Analgetikanephropathie und renal-tubuläre Azidose zu verstehen. Weiterhin Röntgen-Kontrastmittel- sowie Medikamenten-induzierte chronische interstitielle Nierenerkrankungen, Metabolisches Syndrom und Dyslipidämie. Die vorliegende Erfindung umfasst auch die Verwendung der erfindungsgemäßen Verbindungen zur Behandlung und/ oder Prophylaxe von Folgeerscheinungen einer Niereninsuffizienz, wie beispielsweise Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Weiterhin eignen sich die erfindungsgemäßen Salze auch zur Behandlung und/oder Prophylaxe von pulmonaler arterieller Hypertonie (PAH) und anderen Formen der pulmonalen Hypertonie (PH), der chronisch-obstruktive Lungenerkrankung (COPD), des akuten Atemwegssyndrom (ARDS), der akuten Lungenschädigung (ALI), der alpha-1-Antitrypsin-Defizienz (AATD), der Lungenfibrose, des Lungenemphysem (z.B. durch Zigarettenrauch induziertes Lungenemphysem), der zystischer Fibrose (CF), von akutem Koronarsyndrom (ACS), Herzmuskelentzündungen (Myokarditis) und anderen autoimmune Herzerkrankungen (Perikarditis, Endokarditis, Valvolitis, Aortitis, Kardiomyopathien), kardiogenem Schock, Aneurysmen, Sepsis (SIRS), multiplem Organversagen (MODS, MOF), entzündlichen Erkrankungen der Niere, chronischen Darmentzündungen (IBD, Crohn's Disease, UC), Pankreatitis, Peritonitis, rheumatoiden Erkrankungen, entzündlichen Hauterkrankungen sowie entzündlichen Augenerkrankungen.

Die erfindungsgemäßen Salze können weiterhin verwendet werden zur Behandlung und/ oder Prophylaxe von asthmatischen Erkrankungen unterschiedlicher Schweregrade mit intermittierendem oder persistierendem Verlauf (refraktives Asthma, bronchiales Asthma, allergisches Asthma, intrinsisches Asthma, extrinsisches Asthma, durch Medikamente oder durch Staub induziertes Asthma), von verschiedenen Formen der Bronchitis (chronische Bronchitis, infektiöse Bronchitis, eosinophile Bronchitis), von Bronchiolitis obliterans, Bronchiektasie, Pneumonie, idiopathischer interstitieller Pneumonie, Farmerlunge und verwandten Krankheiten, Husten- und Erkältungskrankheiten (chronischer entzündlicher Husten, iatrogener Husten), Nasenschleimhautentzündungen (einschließlich medikamentöse Rhinitis, vasomotorische Rhinitis und jahreszeitabhängige, allergische Rhinitis, z.B. Heuschnupfen) und von Polypen.

Weiterhin sind die erfindungsgemäßen Salze zur Behandlung und/oder Prophylaxe fibrotischer Erkrankungen der inneren Organe, wie beispielsweise der Lunge, des Herzens, der Niere, des Knochenmarks und insbesondere der Leber, sowie dermatologischer Fibrosen und fibrotischer Erkrankungen des Auges, geeignet. Im Sinne der vorliegenden Erfindungen umfasst der Begriff fibrotischer Erkrankungen insbesondere die folgenden Begriffe Leberfibrose, Leberzirrhose, Lungenfibrose, Endomyocardfibrose, Kardiomyopathie, Nephropathie, Glomerulonephritis, interstitielle Nierenfibrose, fibrotische Schäden in Folge von Diabetes, Knochenmarksfibrose und ähnliche fibrotische Erkrankungen, Skleroderma, Morphaea, Keloide, hypertrophe Narbenbildung (auch nach chirurgischen Eingriffen), Naevi, diabetische Retinopathie und proliferative Vitroretinopathie.

Weiterhin eignen sich die erfindungsgemäßen Salze zur Bekämpfung postoperativer Narbenbildung, z.B. in Folge von Glaukoma-Operationen.

Des Weiteren können die erfindungsgemäßen Salze ebenfalls kosmetisch bei alternder und verhornender Haut eingesetzt werden.

Darüber hinaus können die erfindungsgemäßen Salze auch eingesetzt werden zur Behandlung und/oder Prophylaxe von Dyslipidämien (Hypercholesterolämie, Hypertriglyceridämie, erhöhte Konzentrationen der postprandialen Plasma-Triglyceride, Hypoalphalipoproteinämie, kombinierte Hyperlipidämien), Nephropathie und Neuropathie), Krebserkrankungen (Hautkrebs, Hirntumore, Brustkrebs, Knochenmarktumore, Leukämien, Liposarcome, Karzinome des Magen-Darm-Traktes, der Leber, Bauchspeicheldrüse, Lunge, Niere, Harnleiter, Prostata und des Genitaltraktes sowie bösartige Tumore des lymphoproliferativen Systems wie z.B. Hodgkin's und Non-Hodgkin's Lymphom), von Erkrankungen des Gastrointestinaltraktes und des Abdomen (Glossitis, Gingivitis, Periodontitis, Oesophagitis, eosinophile Gastroenteritis, Mastocytose, Morbus Crohn, Colitis, Proctitis, Pruritis ani, Diarrhöe, Zöliakie, Hepatitis, chronischer Hepatitis, Leberfibrose, Leberzirrhose, Pankreatitis und Cholecystitis), Hauterkrankungen (allergische Hauterkrankungen, Psoriasis, Akne, Ekzeme, Neurodermitis, vielfältige Formen der Dermatitis, sowie Keratitis, Bullosis, Vasculitis, Cellulitis, Panniculitis, Lupus erythematodes, Erythema, Lymphome, Hautkrebs, Sweet-Syndrom, Weber-Christian-Syndrom, Narbenbildung, Warzenbildung, Frostbeulen), von Erkrankungen des Skelettknochens und der Gelenke sowie der Skelettmuskel (vielfältige Formen der Arthritis, vielfältige Formen der Arthropathien, Sklerodermia sowie von weiteren Erkrankungen mit einer entzündlichen oder immunologischen Komponente, wie beispielsweise paraneoplastisches Syndrom, bei Abstoßungsreaktionen nach Organtransplantationen und zur Wundheilung und Angiogenese insbesondere bei chronischen Wunden.

Die erfindungsgemäßen Salze eignen sich weiterhin zur Behandlung und/oder Prophylaxe von ophthalmologischen Erkrankungen wie beispielsweise Glaukom, normotensivem Glaukom, Augenhochdruck und deren Kombinationen, von altersbedingter Makuladegeneration (AMD), trockener oder nicht-exsudativer AMD, feuchter oder exsudativer oder neovaskulärer AMD, choroidaler Neovascularization (CNV), Netzhautablösung, diabetischer Retinopathie, atrophischen Veränderungen des retinalen Pigmentepithels (RPE), hypertrophischen Veränderungen des retinalen Pigmentepithels (RPE), diabetischem Makulaödem, Netzhautvenenverschluss, choroidalem Netzhautvenenverschluss, Makulaödem, Makulaödem aufgrund von Netzhautvenenverschluss, Angiogenese an der Vorderseite des Auges wie kornealer Angiogenese beispielsweise nach Keratitis, Hornhauttransplantation oder Keratoplastik, korneale Angiogenese aufgrund von Hypoxie (extensives Tragen von Kontaktlinsen), Pterygium conjunctivae, subretinalem Ödem und intraretinalem Ödem.

Desweiteren eignen sich die erfindungsgemäßen Salze zur Behandlung und/oder Prophylaxe von erhöhten und hohem Augeninnendruck als Folge von traumatischem Hyphaema, periorbitalem Ödem, postoperativer viscoelastischer Retention, intraokularer Entzündung, Anwendung von Kortikosteroiden, Pupillarblock oder idiopathischen Ursachen sowie von erhöhtem Augeninnendruck nach Trabekulektomie und aufgrund von präoperativen Zusätzen.

Weiterer Gegenstand der vorliegenden Erfindung ist die Verwendung der erfindungsgemäßen Salze zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Salze zur Herstellung eines Arzneimittels zur Behandlung und/oder Prophylaxe von Erkrankungen, insbesondere der zuvor genannten Erkrankungen.

Weiterer Gegenstand der vorliegenden Erfindung sind die erfindungsgemäßen Salze zur Verwendung in einem Verfahren zur Behandlung und/oder Prophylaxe von Herzinsuffizienz, pulmonaler Hypertonie, chronischobstruktiver Lungenerkrankung, Asthma, Niereninsuffizienz, Nephropathien, fibrotischen Erkrankungen der inneren Organe und dermatologischen Fibrosen.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Salze können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Salze in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophilisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. inhalativ, intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer, Aerosole), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale, die intravenöse und die inhalative Applikation.

Die erfindungsgemäßen Salze können in die angeführten Applikationsformen überführt werden. Dies kann in an sich bekannter Weise durch Mischen mit inerten, nicht-toxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Weiterer Gegenstand der Erfindung ist ein Verfahren zur Herstellung der erfindungsgemäßen Salze indem man die Verbindung der Formel (I) in Form der freien Säure beispielsweise in einem inerten Lösungsmittel löst (optional kann ein Cosolvens zugegeben werden) und mit einer Lösung der salzbildenden Base bei einer Temperatur von 10 °C bis 60 °C bevorzugt bei 20 °C bis 40 °C, besonders bevorzugt bei 25 °C bzw. bei Raumtemperatur rührt oder schüttelt. Die erhaltenen Kristalle der Salze werden abgetrennt und zur Entfernung des vorhandenen Lösungsmittels bei Raumtemperatur oder bei erhöhter Temperatur bis zur Gewichtskonstanz getrocknet.

Als inerte Lösungsmittel eignen sich niedere Alkohole, wie Methanol, Ethanol, n-Propanol, iso-Propanol, n-Butanol, sec-Butanol, iso-Butanol, 1-Pentanol, Ketone wie Aceton, Alkane, wie n-Pentan, Cyclopentan, n-Hexan, Cyclohexan, oder Tetrahydrofuran, Acetonitril, Toluol, Ethylacetat, 1,4-Dioxan oder Gemische der genannten Lösungsmittel. Bevorzugt sind Acetonitril, Toluol und Isopropanol oder Gemische der genannten Lösungsmittel.

Gegebenenfalls kann ein Cosolvens verwendet werden. Hierfür eignen sich Acetonitril, Aceton, 2-Propanol, Isopropylacetat, 2-Methyltetrafuran, Toluol, 1,4-Dioxan oder auch Gemische davon. Abhängig von der verwendeten Salz bildenden Base sind Toluol, Isopropylacetat oder Acetonitril bevorzugt.

Als Salz bildende Basen eignen sich grundsätzlich Natriumhydroxid, Kaliumhydroxid, Cholinbicarbonat, Ammoniumcarbonat, Natriumcarbonat, Kaliumcarbonat, L-Lysin, Tris(hydroxymethyl)aminomethan, N-Methyl-D-Glucamin, L-Arginin, Natriumbicarbonat oder Kaliumbicarbonat. Erfindungsgemäß haben sich L-Lysin, Natriumbicarbonat und Kaliumbicarbonat als besonders gut zur Salzbildung geeignet erwiesen.

Generell werden die Herstellungsprozesse unter Atmosphärendruck ausgeführt. Jedoch ist es auch möglich bei einem erhöhtem oder erniedrigtem Druck, beispielsweise von 0.5 bis 5 bar, zu arbeiten.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### Experimenteller Teil

Die Röntgendiffraktogramme wurden mit einem XRD-Transmissions/Reflexions-Diffraktometer X'Pert PRO (PANalytical) bei Raumtemperatur aufgenommen (Strahlung: Kupfer, Kα1, Wellenlänge:1,5406 Å). Es gab keine Probenvorbereitung.

### Ausführungsbeispiele

### Herstellung der Verbindung der Formel (I) (freie Säure)

### (S)-4-Trifluormethyl-indan-1-ol

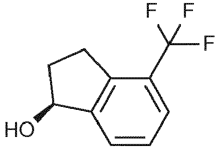

Eine Lösung von 55.7 g (278.3 mmol) 4-Trifluormethyl-1-indanon, 194 ml (1.391 mol) Triethylamin und 1.60 g (2.50 mmol) RuCl(p-cymene)[(S,S)-TsDPEN] (CAS-Nr: 192139-90-5; IUPAC-Name: (S,S)-N-(p-Toluolsulfonyl)-1,2-diphenylethandiamin(chlor)[1-methyl-4-(propan-2-yl)benzol]-ruthenium(II)) in 258 ml Dichlormethan wurde unter Argon auf 35°C erwärmt und bei dieser Temperatur langsam mit 52.5 ml (1.391 mol) Ameisensäure versetzt (Zugabezeit ca. 40 min). Die Temperatur der Reaktionsmischung stieg dabei auf 42°C. Nach vollständiger Zugabe wurde die Mischung weitere 2 h bei 38°C gerührt. Alle flüchtigen Bestandteile wurden am Rotationsverdampfer und im HV entfernt. Anschließend wurde der Rückstand in wenig Dichlormethan gelöst und über 1 kg Kieselgel gereinigt (Eluens: zuerst 3 Liter Cyclohexan / Essigsäureethylester 5:1 dann 6 Liter Cyclohexan / Essigsäureethylester 1:1). Die geeigneten Fraktionen wurden am Rotationsverdampfer eingeengt und das Produkt im HV getrocknet. Man erhielt 51.2 g (90 % d. Th.) der Titelverbindung.

¹H-NMR (400MHz, DMSO-d₆): δ [ppm]= 1.76 - 1.91 (m, 1H), 2.40 (ddt, 1H), 2.86 (dt, 1H), 3.01 - 3.13 (m, 1H), 5.09 (q, 1H), 5.45 (d, 1H), 7.38 - 7.48 (m, 1H), 7.55 (d, 1H), 7.62 (d, 1H). Chirale analytische HPLC (Methode 25): Rₜ = 7.49 min; 99 % ee

### Ethyl-1-(3-methyl-2-oxo-2,3-dihydro-1,3-benzoxazol-6-yl)-2,4-dioxo-1,2,3,4-tetrahydropyrimidin-5-carboxylat

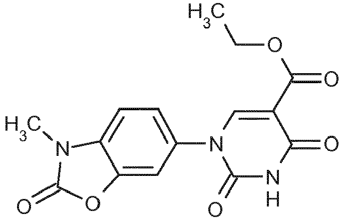

40.0 g (243.7 mmol) 6-Amino-3-methyl-1,3-benzoxazol-2(3H)-on wurden in 2.5 L Ethanol vorgelegt und mit 63.2 g (243.7 mmol) Ethyl-3-ethoxy-2-[(ethoxycarbonyl)carbamoyl]acrylat (Darstellung siehe: Senda, Shigeo; Hirota, Kosaku; Notani, Jiyoji, Chemical & Pharmaceutical Bulletin (1972), 20(7), 1380-8) versetzt. Nach einigen Minuten bildete sich eine dicke Suspension. Diese Mischung wurde 1.5 h auf Rückflusstemperatur erhitzt. Nach leichtem Abkühlen (ca. 60°C) wurden 27.3 g (243.7 mmol) Kalium-*tert.*-butylat zugegeben und das Reaktionsgemisch 4.5 h bei Rückflusstemperatur weiter gerührt. Zur Aufarbeitung wurde die Reaktionssuspension leicht abgekühlt (ca. 60°C) dann in 10 Liter kalte 1N Salzsäure eingerührt. Der Feststoff wurde abgesaugt, mit Wasser gewaschen und im Vakuum-Trockenschrank bei 70°C über Nacht getrocknet. Man erhielt 64.0 g (79 % d. Th.) der Titelverbindung.
LC-MS (Methode 1: Rₜ = 0.59 min; MS (ESIpos): m/z = 332 (M+H)⁺.
¹H-NMR (400MHz, DMSO-d₆): δ [ppm] = 1.22 (t, 3H), 3.38 (s, 3H), 4.17 (q, 2H), 7.38 (s, 2H), 7.59 (s, 1H), 8.26 (s, 1H), 11.69 (s, 1H).

### Ethyl-1-(3-methyl-2-oxo-2,3-dihydro-1,3-benzoxazol-6-yl)-2,4-dioxo-3-[(1R)-4-(trifluormethyl)-2,3-dihydro-1H-inden-1-yl]-1,2,3,4-tetrahydropyrimidin-5-carboxylat (R-Enantiomer)

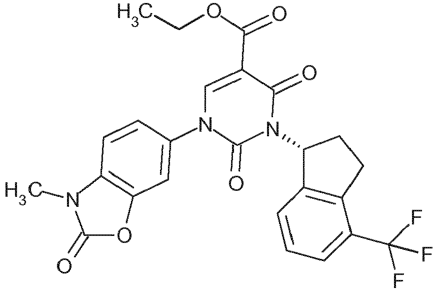

*Methode A*: Eine Lösung von 200 mg (0.60 mmol) Ethyl-1-(3-methyl-2-oxo-2,3-dihydro-1,3-benzoxazol-6-yl)-2,4-dioxo-1,2,3,4-tetrahydropyrimidin-5-carboxylat (siehe oben) und 475 mg (1.81 mmol) Triphenylphosphin wurde unter Argon in THF/DMF 1:1 (7.6 ml) auf -30°C abgekühlt. 238 µl (1.20 mmol) Diisopropylazodicarboxylat wurden zugetropft und anschließend eine Lösung von 146 mg (0.69 mmol (1S)-4-(Trifluormethyl)-indan-1-ol (siehe oben) in ca. 1 ml THF zugetropft. Die Reaktionsmischung wurde auf Raumtemperatur erwärmt und 30 min bei Raumtemperatur gerührt. Zur Aufarbeitung wurde das Gemisch auf 0°C abgekühlt, mit 5 ml 1M Salzsäure versetzt, auf Raumtemperatur erwärmt und 30 min gerührt. Anschließend wurde mit Essigsäureethylester extrahiert. Die organische Phase wurde zweimal mit 1M Salzsäure und einmal mit gesättigter Natriumchlorid-Lösung gewaschen, über Magnesiumsulfat getrocknet und im Vakuum eingeengt. Der Rückstand wurde mit Ethanol ausgerührt, der ausgefallene Feststoff abgesaugt und verworfen. Das Filtrat wurde eingeengt, in wenig Dichlormethan gelöst und mittels Flashchromatographie (Dichlormethan/Methanol 120:1→ 20:1) gereinigt. Man erhielt 135 mg (43 % d. Th.) der Titelverbindung in ca. 95 %-iger Reinheit.
LC-MS (Methode 1): Rₜ = 1.13 min; m/z = 516 (M+H)⁺.
¹H-NMR (400MHz, DMSO-d₆): δ [ppm] = 1.22 (t, 3H), 2.37 - 2.43 (m, 1H), 2.43 - 2.48 (m, 1H, telweise verdeckt durch DMSO-Signal), 3.03 - 3.14 (m, 1H), 3.22 - 3.30 (m, 1H, teilweise verdeckt durch Wasser-Signal), 3.38 (s, 3H), 4.18 (q, 2H), 6.34 - 6.56 (m, 1H), 7.32 - 7.43 (m, 3H), 7.45 - 7.50 (m, 1H), 7.53 (d, 1H), 7.55 - 7.64 (m, 1H), 8.35 (s, 1H).

In einem analogen Versuch konnte eine Fraktion mit 99 %-iger Reinheit isoliert werden. Für diese Charge wurde der spezifische optische Drehwert gemessen:
Spezifischer optischer Drehwert: α_{D}²⁰ = +132.9°, (Chloroform, c = 0.395 g/100 ml).

*Methode B:* Eine Lösung von 5.0 g (15.1 mmol) Ethyl-1-(3-methyl-2-oxo-2,3-dihydro-1,3-benzoxazol-6-yl)-2,4-dioxo-1,2,3,4-tetrahydropyrimidin-5-carboxylat (siehe oben), 6.73 g (25.7 mmol) Triphenylphosphin und 3.66 g (18.1 mmol) (1S)-4-(Trifluormethyl)indan-1-ol (siehe oben) wurde unter Argon in 240 ml DMF/THF 2:1 (v/v) vorgelegt und auf -15°C abgekühlt. 4.76 ml (24.15 mmol) Diisopropylazodicarboxylat wurden langsam so zugetropft, dass die Temperatur der Reaktionsmischung nicht über -10°C anstieg. Am Ende der Zugabe wurde die Mischung noch 1 h bei -10°C gerührt, dann auf Raumtemperatur erwärmt und auf 1.3 L Wasser gegossen. Das Gemisch wurde zweimal mit je 300 ml Essigsäureethylester extrahiert. Die vereinigten organischen Phasen wurden mit einer gesättigten Natriumchloridlösung gewaschen, über Magnesiumsulfat getrocknet und am Rotationsverdampfer vom Lösungsmittel befreit. Der Rückstand (18 g) wurde in zwei chromatographischen Schritten gereinigt: zuerst über eine 200 g Kieselgel-Säule mit Dichlormethan / Aceton 97.5 : 2.5 als Eluent. Die erhaltenen produkthaltigen Fraktionen wurden eingeengt und der Rückstand erneut über eine 200 g Kieselgel-Säule appliziert. Mit 2.5 L Cyclohexan/ Essigsäureethylester 1:1 als Eluent wurden weitere Verunreinigungen eluiert, dann wurde das gewünschte Produkt mit Dichlormethan / Methanol 95 : 5 aus der Säule eluiert. Man erhielt so 3.40 g (44 % d. Th.) der Titelverbindung in 95 %-iger Reinheit (NMR zeigte ca. 5 % Essigsäureethylester). Weitere 920 mg konnten durch erneute Reinigung einer Mischfraktion erhalten werden. Gesamtausbeute: 4.32 g (56 % d. Th.).
LC-MS (Methode 1): Rₜ = 1.15 min; m/z = 516 (M+H)⁺.
¹H-NMR (400MHz, CD₂Cl₂): δ [ppm] = 1.31 (t, 3H), 2.37 - 2.49 (m, 1H), 2.59 (dtd, 1H), 3.14 (dt, 1H), 3.40 (s, 3H), 3.42 - 3.53 (m, 1H), 4.29 (q, 2H), 6.54 - 6.68 (m, 1H), 7.06 (d, 1H), 7.17 (d, 1H), 7.22 (s, 1H), 7.26 - 7.36 (m, 2H), 7.49 (d, 1H), 8.28 (s, 1H).

### 1-(3-Methyl-2-oxo-2,3-dihydro-1,3-benzoxazol-6-yl)-2,4-dioxo-3-[(1R)-4-(trifluormethyl)-2,3-dihydro-1H-inden-1-yl]-1,2,3,4-tetrahydropyrimidin-5-carbonsäure (R-Enantiomer)

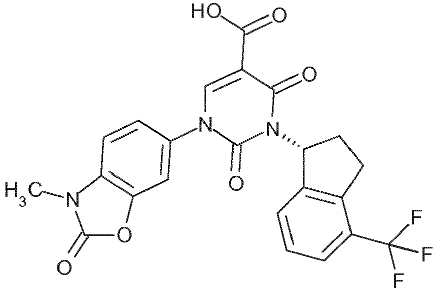

3.40 g (6.60 mmol) von Ethyl-1-(3-methyl-2-oxo-2,3-dihydro-1,3-benzoxazol-6-yl)-2,4-dioxo-3-[(1R)-4-(trifluonnethyl)-2,3-dihydro-1H-inden-1-yl]-1,2,3,4-tetrahydropyrimidin-5-carboxylat (siehe oben) wurden in 44 ml Eisessig und 22 ml konzentrierter Salzsäure 1 h bei Rückflusstemperatur gerührt. Nach leichter Abkühlung (ca. 60 °C) wurde das Gemisch vollständig im Vakuum eingeengt. Der amorphe Rückstand wurde mit 50 ml Isopropanol versetzt und 15 min zum Rückfluss erhitzt, wobei sich ein Feststoff bildete. Die Suspension wurde anschließend auf 10°C gekühlt und dann der Feststoff abgesaugt. Der Feststoff wurde zweimal mit je 15 ml Isopropanol gewaschen, abgesaugt und im Hochvakuum getrocknet. Man erhielt 2.53 g (79 % d. Th.) der Titelverbindung.
LC-MS (Methode 1): Rₜ = 1.12 min; m/z = 488 (M+H)⁺.
Chirale analytische HPLC (Methode 14): Rₜ= 13.3 min; ca. 99 % ee

¹H-NMR (400MHz, CD₂Cl₂): δ [ppm]= 2.40 - 2.52 (m, 1H), 2.59 - 2.72 (m, 1H), 3.12 - 3.25 (m, 1H), 3.41 (s, 3H), 3.44 - 3.56 (m, 1H), 6.58 - 6.69 (m, 1H), 7.04 - 7.11 (m, 1H), 7.15 - 7.21 (m, 1H), 7.24 (br.s, 1H), 7.29 - 7.38 (m, 2H), 7.53 (s, 1H), 8.54 (s, 1H), 12.39 (br. s, 1H).
Spezifischer Drehwert α_{D}²⁰ = +135.3° (Methanol, c = 0.43).
In einem analogen Experiment wurde der spezifischer Drehwert des Produkts in Chloroform gemessen: α_{D}²⁰ = +159.5° (Chloroform, c = 0.395).
Eine Röntgenstrulcturanalyse im Komplex mit Chymase bestätigte für dieses Enantiomer die R-Konfiguration.

### Beispiel 1

### Herstellung des L-Lysinsalzes von 1-(3-Methyl-2-oxo-2,3-dihydro-1,3-benzoxazol-6-yl)-2.4-dioxo-3-[(1R)-4-(trifluormethyl)-2,3-dihydro-1H-inden-1-yl]-1,2,3,4-tetrahydropyrimidin-5-carbonsäure

Ca. 300 mg 1-(3-Methyl-2-oxo-2,3-dihydro-1,3-benzoxazol-6-yl)-2,4-dioxo-3-[(1R)-4-(trifluormethyl)-2,3-dihydro-1H-inden-1-y1]-1,2,3,4-tetrahydropyimidin-5-carbonsäure (freie Säure) wurden in 30 ml Acetonitril gelöst. Unter Schwenken wurden 30 ml Toluol als Cosolvens zugegeben. Anschließend wurde mit einer Lösung aus 90 mg L-Lysin in 10 ml Wasser versetzt und bei Raumtemperatur über Nacht gerührt. Anschließend wurde die Suspension filtriert und der Rückstand bei Raumtemperatur bei Umgebungsfeuchte getrocknet. Er wurde röntgendiffraktometrisch untersucht und entspricht der Titelverbindung.

### Beispiel 2

### Herstellung des Natriumsalzes von 1-(3-Methyl-2-oxo-2,3-dihydro-1,3-benzoxazol-6-yl)-2,4-dioxo-3-[(1R)-4-(trifluormethyl)-2,3-dihydro-1H-inden-1-yl]-1,2,3,4-tetrahydropyrimidin-5-carbonsäure

Ca. 300 mg 1-(3-Methyl-2-oxo-2,3-dihydro-1,3-benzoxazol-6-yl)-2,4-dioxo-3-[(1R)-4-(trifluormethyl)-2,3-dihydro-1H-inden-1-yl]-1,2,3,4-tetrahydropyrimidin-5-carbonsäure (freie Säure) wurden in 30 ml Acetonitril gelöst. Unter Schwenken wurden 30 ml Isopropylacetat als Cosolvens zugegeben. Anschließend wurde mit einer Lösung aus 65,2 mg Natriumbicarbonat in 10 ml Wasser versetzt und bei Raumtemperatur für 60 min. gerührt. Anschließend wurde die Suspension filtriert und der Rückstand bei Raumtemperatur bei Umgebungsfeuchte getrocknet. Er wurden röntgendiffraktometrisch untersucht und entspricht der Titelverbindung .

### Beispiel 3

### Herstellung des Kaliumsalzes von 1-(3-Methyl-2-oxo-2,3-dihydro-1,3-benzoxazol-6-yl)-2,4-dioxo-3-[(1R)-4-(trifluormethyl)-2,3-dihydro-1H-inden-1-yl]-1,2,3,4-tetrahydropyrimidin-5-carbonsäure

Ca. 300 mg 1-(3-Methyl-2-oxo-2,3-dihydro-1,3-benzoxazol-6-yl)-2,4-dioxo-3-[(1R)-4-(trifluormethyl)-2,3-dihydro-1H-inden-1-yl]-1,2,3,4-tetrahydropyrimidin-5-carbonsäure (freie Säure) wurden in 30 ml Acetonitril gelöst. Unter Schwenken wurden weitere 30 ml Acetonitril zugegeben. Anschließend wurde mit einer Lösung aus 85,1 mg Kaliumbicarbonat in 10 ml Wasser versetzt und bei Raumtemperatur für 60 min. gerührt. Anschließend wurde die Suspension filtriert und der Rückstand bei Raumtemperatur bei Umgebungsfeuchte getrocknet. Er wurde röntgendiffraktometrisch untersucht und entspricht der Titelverbindung.

**Tabelle 1: Röntgendiffraktometrie der freien Säure von 1-(3-Methyl-2-oxo-2,3-dihydro-1,3-benzoxazol-6-yl)-2, 4-dioxo-3-[(1R)-4-(trifluormethyl)-2,3-dihydro-1H-inden-1-yl]-1,2,3,4-tetrahydropyrimidin-5-carbonsäure sowie deren Salze**

| **Peakmaximum [2 Theta]** | | |
|---|---|---|
| **Lysin-Salz** | **Natriumsalz** | **Kaliumsalz** |
| 6.1 | 3.6 | 6.0 |
| 9.9 | 4.3 | 6.5 |
| 10.9 | 5.3 | 9.4 |
| 12.2 | 5.9 | 10.4 |
| 14.1 | 6.0 | 11.2 |
| 14.9 | 7.2 | 12.0 |
| 16.2 | 8.0 | 13.0 |
| 16.7 | 8.6 | 15.3 |
| 16.9 | 9.0 | 16.5 |
| 18.5 | 9.6 | 16.8 |
| 18.7 | 10.6 | 18.0 |
| 19.2 | 10.9 | 18.5 |
| 20.0 | 11.3 | 19.2 |
| 21.6 | 11.8 | 19.8 |
| 22.3 | 12.8 | 20.5 |
| 22.7 | 13.0 | 21.1 |
| 23.0 | 13.5 | 21.7 |
| 24.4 | 14.1 | 22.7 |
| 24.4 | 14.5 | 23.7 |
| 24.8 | 15.5 | 24.2 |
| 25.7 | 16.0 | 25.2 |
| 26.9 | 17.1 | 27.3 |
| 27.1 | 17.6 | 28.2 |
| 27.8 | 17.9 | 28.8 |
| 29.5 | 18.1 | 30.0 |
| 30.1 | 18.6 | 31.2 |
| 30.3 | 18.9 | 31.5 |
| 30.9 | 19.1 | 34.0 |
| 31.4 | 19.6 | 36.1 |
| 32.1 | 20.3 | |
| 33.1 | 20.9 | |
| 33.4 | 21.6 | |
| 33.8 | 22.0 | |
| 34.2 | 22.5 | |
| 35.0 | 23.5 | |
| 35.6 | 23.8 | |
| 36.1 | 24.3 | |
| 37.0 | 24.7 | |
| 37.5 | 25.1 | |
| | 25.8 | |
| | 27.1 | |
| | 27.8 | |
| | 28.5 | |
| | 29.0 | |
| | 29.1 | |
| | 30.1 | |
| | 30.4 | |
| | 30.8 | |
| | 31.7 | |

### Kurze Beschreibung der Abbildungen

**Abbildung 1****:** Röntgendiffraktogramm des L-Lysinsalzes von 1-(3-Methyl-2-oxo-2,3-dihydro-1,3-benzoxazol-6-yl)-2,4-dioxo-3-[(1R)-4-(trifluormethyl)-2,3-dihydro-1H-inden-1-yl]-1,2,3,4-tetrahydropyrimidin-5-carbonsäure
**Abbildung 2****:** Röntgendiffraktogramm des Natriumsalzes von 1-(3-Methyl-2-oxo-2,3-dihydro-1,3-benzoxazol-6-yl)-2,4-dioxo-3-[(1R)-4-(trifluormethyl)-2,3-dihydro-1H-inden-1-yl]-1,2,3,4-tetrahydropyrimidin-5-carbonsäure
**Abbildung 3****:** Röntgendiffraktogramm des Kaliumsalzes von 1-(3-Methyl-2-oxo-2,3-dihydro-1,3-benzoxazol-6-yl)-2,4-dioxo-3-[(1R)-4-(trifluormethyl)-2,3-dihydro-1H-inden-1-yl]-1,2,3,4-tetrahydropyrimidin-5-carbonsäure

## Patentansprüche

1. Salze der 1-(3-Methyl-2-oxo-2,3-dihydro-1,3-benzoxazol-6-yl)-2,4-dioxo-3-[(1R)-4-(trifluormethyl)-2,3-dihydro-1H-inden-1-yl]-1,2,3,4-tetrahydropyrimidin-5-carbonsäure der Formel (I)

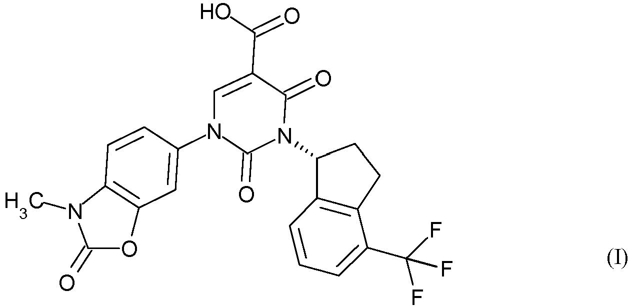

2. Salze gemäß Anspruch 1, **dadurch gekennzeichnet, dass** es sich um Aminosäuresalze und Alkalimetallsalze der 1-(3-Methyl-2-oxo-2,3-dihydro-1,3-benzoxazol-6-yl)-2,4-dioxo-3-[(1R)-4-(trifluormethyl)-2,3-dihydro-1H-inden-1-yl]-1,2,3,4-tetrahydropyrimidin-5-carbonsäure handelt.

3. Salz gemäß Anspruch 1 oder 2, **dadurch gekennzeichnet, dass** es sich um das Lysinsalz der 1-(3-Methyl-2-oxo-2,3-dihydro-1,3-benzoxazol-6-yl)-2,4-dioxo-3-[(1R)-4-(trifluormethyl)-2,3-dihydro-1H-inden-1-yl]-1,2,3,4-tetrahydropyrimidin-5-carbonsäure handelt.

4. Salz gemäß Anspruch 1, 2 oder 3, **dadurch gekennzeichnet, dass** es sich um das L-Lysinsalz der 1-(3-Methyl-2-oxo-2,3-dihydro-1,3-benzoxazol-6-yl)-2,4-dioxo-3-[(1R)-4-(trifluormethyl)-2,3 - dihydro-1H-inden-1-yl]-1,2,3,4-tetrahydropyrimidin-5-carbonsäure handelt.

5. Salz gemäß Anspruch 4, **dadurch gekennzeichnet, dass** das Röntgendiffraktogramm der Verbindung ein Peakmaximum des 2 Theta Winkels bei 16.9 zeigt.

6. Salz gemäß Anspruch 4 oder 5, **dadurch gekennzeichnet, dass** das Röntgendiffraktogramm der Verbindung Peakmaxima des 2 Theta Winkels bei 16.9, 22.3 und 20.0 zeigt.

7. Salz gemäß Anspruch 1 oder 2, **dadurch gekennzeichnet, dass** es sich um das Natriumsalz der 1-(3-Methyl-2-oxo-2,3-dihydro-1,3-benzoxazol-6-yl)-2,4-dioxo-3-[(1R)-4-(trifluormethyl)-2,3-dihydro-1H-inden-1-yl]-1,2,3,4-tetrahydropyrimidin-5-carbonsäure handelt.

8. Salz gemäß Anspruch 7, **dadurch gekennzeichnet, dass** das Röntgendiffraktogramm der Verbindung ein Peakmaximum des 2 Theta Winkels bei 17.6 zeigt.

9. Salz gemäß Anspruch 7 oder 8, **dadurch gekennzeichnet, dass** das Röntgendiffraktogramm der Verbindung Peakmaxima des 2 Theta Winkels bei 17.6, 17.9 und 19.1 zeigt.

10. Salz gemäß Anspruch 1 oder 2, **dadurch gekennzeichnet, dass** es sich um das Kaliumsalz der 1-(3-Methyl-2-oxo-2,3-dihydro-1,3-benzoxazol-6-yl)-2,4-dioxo-3-[(1R)-4-(trifluormethyl)-2,3-dihydro-1H-inden-1-yl]-1,2,3,4-tetrahydropyrimidin-5-carbonsäure handelt.

11. Salz gemäß Anspruch 10, **dadurch gekennzeichnet, dass** das Röntgendiffraktogramm der Verbindung ein Peakmaximum des 2 Theta Winkels bei 23.7 zeigt.

12. Salz gemäß Anspruch 10 oder 11, **dadurch gekennzeichnet, dass** das Röntgendiffraktogramm der Verbindung Peakmaxima des 2 Theta Winkels bei, 23.7, 15.3und 20.5 zeigt.

13. Salz nach einem der Ansprüche 1 bis 12 zur Behandlung von Krankheiten.

14. Arzneimittel enthaltend eine Verbindung gemäß einem der Ansprüche 1 bis 12 in mehr als 90 Gewichtsprozente bezogen auf die Gesamtmenge der enthaltenen Verbindung der Formel (I).

15. Arzneimittel enthaltend eine Verbindung gemäß einem der Ansprüche 1 bis 12 in mehr als 95 Gewichtsprozente bezogen auf die Gesamtmenge der enthaltenen Verbindung der Formel (I).

16. Verfahren zur Herstellung der Verbindung gemäß einem der Ansprüche 1 bis 12, indem man die Verbindung der Formel (I) in Form der freien Säure in einem inerten Lösungsmittel löst und mit einer Lösung der salzbildenden Base bei einer Temperatur von 10 °C bis 60 °C rührt oder schüttelt.

17. Verwendung der Verbindung gemäß einem der Ansprüche 1 bis 12 zur Herstellung eines Arzneimittels zur Behandlung und/oder Prophylaxe kardiovaskulärer, entzündlicher, allergischer und/oder fibrotischer Erkrankungen.

## Claims

1. Salts of 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid of the formula (I)

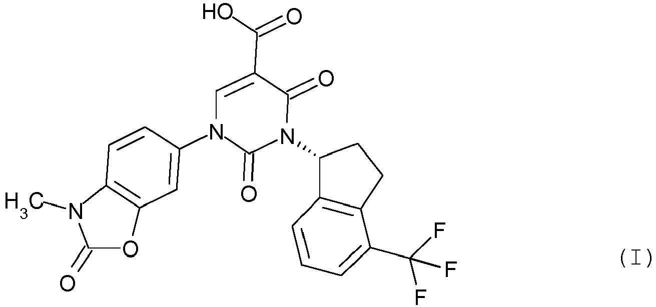

2. Salts according to Claim 1, **characterized in that** they are amino acid salts and alkali metal salts of 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid.

3. Salt according to Claim 1 or 2, **characterized in that** it is the lysine salt of 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid.

4. Salt according to Claim 1, 2 or 3, **characterized in that** it is the L-lysine salt of 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid.

5. Salt according to Claim 4, **characterized in that** the X-ray diffractogram of the compound has a peak maximum of the 2 theta angle at 16.9.

6. Salt according to Claim 4 or 5, **characterized in that** the X-ray diffractogram of the compound has peak maxima of the 2 theta angle at 16.9, 22.3 and 20.0.

7. Salt according to Claim 1 or 2, **characterized in that** it is the sodium salt of 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid.

8. Salt according to Claim 7, **characterized in that** the X-ray diffractogram of the compound has a peak maximum of the 2 theta angle at 17.6.

9. Salt according to Claim 7 or 8, **characterized in that** the X-ray diffractogram of the compound has peak maxima of the 2 theta angle at 17.6, 17.9 and 19.1.

10. Salt according to Claim 1 or 2, **characterized in that** it is the potassium salt of 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid.

11. Salt according to Claim 10, **characterized in that** the X-ray diffractogram of the compound has a peak maximum of the 2 theta angle at 23.7.

12. Salt according to Claim 10 or 11, **characterized in that** the X-ray diffractogram of the compound has peak maxima of the 2 theta angle at 23.7, 15.3 and 20.5.

13. Salt according to any of Claims 1 to 12 for the treatment of disorders.

14. Medicament comprising a compound according to any of Claims 1 to 12 in more than 90 percent by weight based on the total amount of the compound of the formula (I) present.

15. Medicament comprising a compound according to any of Claims 1 to 12 in more than 95 percent by weight based on the total amount of the compound of the formula (I) present.

16. Process for preparing the compound according to any of Claims 1 to 12, which comprises dissolving the compound of the formula (I) in the form of the free acid in an inert solvent and stirring or shaking with a solution of the salt-forming base at a temperature of 10°C to 60°C.

17. Use of the compound according to any of Claims 1 to 12 for producing a medicament for treatment and/or prophylaxis of cardiovascular, inflammatory, allegic and/or fibrotic disorders.

## Revendications

1. Sels de l'acide 1-(3-méthyl-2-oxo-2,3-dihydro-1,3-benzoxazol-6-yl)-2,4-dioxo-3-[(1R)-4-(trifluorométhyl)-2,3-dihydro-1H-indén-1-yl]-1,2,3,4-tétrahydropyrimidine-5-carboxylique de formule (I)

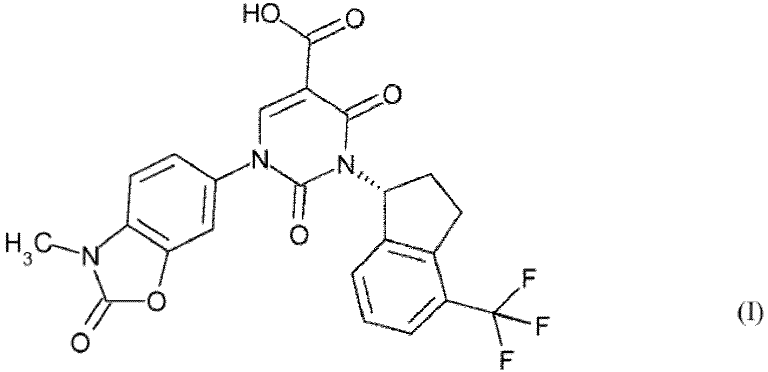

2. Sels selon la revendication 1, **caractérisés en ce qu'**il s'agit de sels d'acides aminés et de sels de métaux alcalins de l'acide 1-(3-méthyl-2-oxo-2,3-dihydro-1,3-benzoxazol-6-yl)-2,4-dioxo-3-[(1R)-4-(trifluorométhyl)-2,3-dihydro-1H-indén-1-yl]-1,2,3,4-tétrahydropyrimidine-5-carboxylique.

3. Sel selon la revendication 1 ou 2, **caractérisé en ce qu'**il s'agit du sel de lysine de l'acide 1-(3-méthyl-2-oxo-2,3-dihydro-1,3-benzoxazol-6-yl)-2,4-dioxo-3-[(1R)-4-(trifluorométhyl)-2,3-dihydro-1H-indén-1-yl]-1,2,3,4-tétrahydropyrimidine-5-carboxylique.

4. Sel selon la revendication 1, 2 ou 3, **caractérisé en ce qu'**il s'agit du sel de L-lysine de l'acide 1-(3-méthyl-2-oxo-2,3-dihydro-1,3-benzoxazol-6-yl)-2,4-dioxo-3-[(1R)-4-(trifluorométhyl)-2,3-dihydro-1H-indén-1-yl]-1,2,3,4-tétrahydropyrimidine-5-carboxylique.

5. Sel selon la revendication 4, **caractérisé en ce que** le diffractogramme de rayon X du composé présente un maximum de pic de l'angle 2-thêta à 16,9.

6. Sel selon la revendication 4 ou 5, **caractérisé en ce que** le diffractogramme de rayon X du composé présente des maxima de pic de l'angle 2-thêta à 16,9, 22,3 et 20,0.

7. Sel selon la revendication 1 ou 2, **caractérisé en ce qu'**il s'agit du sel de sodium de l'acide 1-(3-méthyl-2-oxo-2,3-dihydro-1,3-benzoxazol-6-yl)-2,4-dioxo-3-[(1R)-4-(trifluorométhyl)-2,3-dihydro-1H-indén-1-yl]-1,2,3,4-tétrahydropyrimidine-5-carboxylique.

8. Sel selon la revendication 7, **caractérisé en ce que** le diffractogramme de rayon X du composé présente un maximum de pic de l'angle 2-thêta à 17,6.

9. Sel selon la revendication 7 ou 8, **caractérisé en ce que** le diffractogramme de rayon X du composé présente des maxima de pic de l'angle 2-thêta à 17,6, 17,9 et 19,1.

10. Sel selon la revendication 1 ou 2, **caractérisé en ce qu'**il s'agit du sel de potassium de l'acide 1-(3-méthyl-2-oxo-2,3-dihydro-1,3-benzoxazol-6-yl)-2,4-dioxo-3-[(1R)-4-(trifluorométhyl)-2,3-dihydro-1H-indén-1-yl]-1,2,3,4-tétrahydropyrimidine-5-carboxylique.

11. Sel selon la revendication 10, **caractérisé en ce que** le diffractogramme de rayon X du composé présente un maximum de pic de l'angle 2-thêta à 23,7.

12. Sel selon la revendication 10 ou 11, **caractérisé en ce que** le diffractogramme de rayon X du composé présente des maxima de pic de l'angle 2-thêta à 23,7, 15,3 et 20,5.

13. Sel selon l'une quelconque des revendications 1 à 12 pour le traitement de maladies.

14. Médicament contenant un composé selon l'une quelconque des revendications 1 à 12 à hauteur de plus de 90 pour cent en poids par rapport à la quantité totale du composé de formule (I) contenu.

15. Médicament contenant un composé selon l'une quelconque des revendications 1 à 12 à hauteur de plus de 95 pour cent en poids par rapport à la quantité totale du composé de formule (I) contenu.

16. Procédé de fabrication du composé selon l'une quelconque des revendications 1 à 12, selon lequel le composé de formule (I) sous la forme de l'acide libre est dissous dans un solvant inerte, et agité ou remué avec une solution de la base formant un sel à une température de 10 °C à 60 °C.

17. Utilisation du composé selon l'une quelconque des revendications 1 à 12 pour la fabrication d'un médicament pour le traitement et/ou la prophylaxie de maladies cardiovasculaires, inflammatoires, allergiques et/ou fibrotiques.
